# EUROPEAN PATENT SPECIFICATION

(11) **EP 0 891 962 B1**
(45) Date of publication and mention of the grant of the patent: **18.09.2002**
(21) Application number: 97907369.9
(22) Date of filing: 18.03.1997
(51) Int. Cl.: C07C 45/63, C07C 47/14, C07C 49/16, C07C 49/227, C07C 49/457, C07C 49/687, C07C 49/80, C07C 67/307, C07C 69/716, C07C 69/738, C07C 69/757

(54) **PROCESS FOR THE PREPARATION OF FLUORINATED DICARBONYL COMPOUNDS**
VERFAHREN ZUR HERSTELLUNG FLUORIERTER DICARBONYLVERBINDUNGEN
PROCEDE POUR PREPARER DES COMPOSES DICARBONYLE FLUORES

(30) Priority: 26.03.1996 JP 6995496
(43) Date of publication of application: 20.01.1999
(73) Proprietor: F-TECH, INC., Chuo-ku, Tokyo (JP)
(72) Inventor: NUKUI, Kazunori; Central Research Laboratory,Chic, ; Sakura-shi; Chiba 285 (JP); FUKAMI, Shinji; Central Research Laboratory,Chich, Sakura-shi; Chiba 285 (JP); KAWADA, Kosuke; Central Research Laboratory,Chich, Sakura-shi; Chiba 285 (JP)
(74) Representative: Crump, Julian Richard John
(86) International application number: JP9700881
(87) International publication number: WO97035824

(56) References cited:
- WO-A-94/10120
- CHAMBERS R D ET AL: "Direct Fluorination of 1,3-Dicarbonyl Compounds" TETRAHEDRON, vol. 52, no. 1, 1 January 1996, page 1-8 XP004104578

## Description

### Technical Field

The present invention relates to a novel process for the preparation of fluorinated dicarbonyl compounds useful as intermediates for pesticides, among other applications.

### Background Art

Conventionally, the below-described methods (1) to (6) have been known as processes for the preparation of fluorinated dicarbonyl compounds:
(1) a method in which a β-diketone or a β-ketoester is converted to its metal salt, after which the resultant metal salt is reacted with FClO₃ [Justus Liebigs Ann. Chim., *677*, 9 (1964) or J. Org. Chem., *57*, 2196 (1992)]; with CH₃COOF [J. Org. Chem., *48*, 724 (1983)]; with N-fluoroperfluoropiperidine [J. Fluorine Chem., *52*, 389 (1991)]; with an N-fluoropyridinium salt [J. Am. Chem. Soc., *112*, 8563 (1990) and Japanese Patent Application Laid-Open (*kokai*) No. 62-207228]; or with N-fluorosultam [Tetrahedron Lett., *29*, 6087 (1988) and Helv. Chim. Acta., *72*, 1248 (1989)];
(2) a method in which a β-diketone or a β-ketoester is reacted with a xenon compound (C₁₉XeF₆) [see Tetrahedron Lett., *21*, 277 (1980)]; with CH₃COOF [J. Org. Chem., *48*, 724 (1983)]; with xenon difluoride in the presence of an acid catalyst [J. Chem. Soc., Chem. Commun., *1980,* 759]; with an N-fluoropyridinium salt in the presence or absence of an acid catalyst [J. Am. Chem. Soc., *112*, 8563 (1990) and Japanese Patent Application Laid-Open (*kokai*) No. 62-207228]; or with N-fluorobis (trifluoromethanesulfonyl) amide [J. Chem. Soc., Chem. Commun., *1991,* 179];
(3) a method in which a β-diketone or a β-ketoester is introduced to its corresponding trimethylsilyl enol ether, and the resultant compound is further reacted with fluorine at -78°C [J. Org. Chem., *52*, 4309 (1987)];
(4) a method in which a β-ketoester is chlorinated, and the resultant compound is further reacted with potassium fluoride in the presence of a crown ether for a long time [J. Am. Chem. Soc., *58,* 1803 (1936) and Synthesis, *1977*, 189];
(5) a method for manufacturing a β-diketone or a β-ketoester through a plurality of steps from a perfluoroolefin, such as hexafluoropropene, serving as a starting material [Chem. Lett., 1107 (1980) or Izv. Akad. Nauk, SSSR, Ser, Khim., *1980,* 2827 (CA95-6431z)];
(6) a method in which a β-ketoester is reacted with fluorine gas [J. Org. Chem., *57*, 2196 (1992) or International Patent Publication WO94/10120]; and
(7) a method in which fluorine, diluted with nitrogen, is passed through stirred solutions of 1, 3-diketones and 1, 3-diketoester in formic acid at 10-15°C over 2 hours [Tetrahedron, Vol. 52, No. 1:1-8, 1996].

However, methods (1) and (2) have drawbacks in that there must be used an expensive fluorinating agent which requires intricate processes for synthesis; and that, depending on the reagent, synthesis must be carried out under extremely low reaction temperature and the resultant reagent must be used immediately after preparation because it decomposes at room temperature.

Methods (3) to (5) require multiple reaction steps, and also have the following drawbacks. In method (3), fluorination must be performed at extremely low temperature and the yield is low. In method (4), in addition to the disadvantage that expensive crown ethers must be used, reaction time is long and the reaction yield is very low. Method (5) has the drawbacks that starting materials are expensive and that, due to its unpopularity, the method cannot be said to be a generally applicable method.

Method (6), which is drawn to fluorination of a β-ketoester, requires a large amount of solvent and therefore the size of the employed apparatus increases. The method is extremely disadvantageous as an industrial process in that the yield is so poor that a large amount of raw materials is wasted, and formation of various species of by-products requires cumbersome separation steps.

Method (7) also requires the presence of a large amount of formic acid as solvent and hence a large reaction chamber.

Thus, the above-described methods (1) to (7) are not satisfactory as industrial processes.

Accordingly, an object of the present invention is to provide a simple process which enables production of fluorinated dicarbonyl compounds at high yield.

### Disclosure of the Invention

In view of the foregoing, the present inventors have conducted earnest studies, and have found that fluorinated dicarbonyl compounds can be manufactured at high yield by reacting dicarbonyl compounds and fluorine gas without use of any solvent and in the resence of a specific acid. The present invention was accomplished based on this finding. Accordingly, the present invention provides a process for manufacturing fluorinated dicarbonyl compounds represented by formula (2) 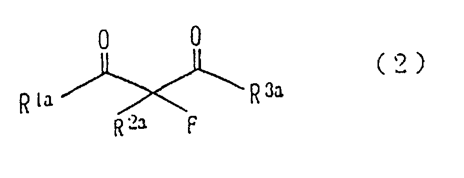 wherein R^{1a} represents a hydrogen atom, a substituted or unsubstituted C₁₋₂₀ alkyl group or a substituted or unsubstituted C₆₋₁₄ aryl group; R^{2a} represents a hydrogen atom, a halogen atom, a substituted or unsubstituted C₁₋₂₀ alkyl group or a substituted or unsubstituted C₆₋₁₄ aryl group; R^{3a} represents a hydrogen atom, a substituted or unsubstituted C₁₋₂₀ alkyl group, a substituted or unsubstituted C₆₋₁₄ aryl group, a substituted or unsubstituted C₁₋₂₀ alkoxy group, or a substituted or unsubstituted C₆₋₁₄ aryloxy group; and at least two of R^{1a}, R^{2a}, and R^{3a} may be combined together to form a portion of a cyclic structure via an optional hetero atom; by reacting dicarbonyl compounds represented by formula (1) 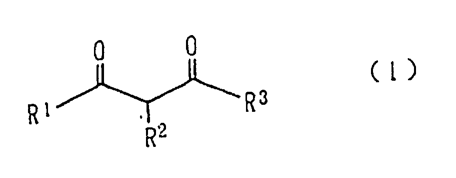 wherein R¹, R², and R³ respectively have the same meanings as R^{1a}, R^{2a}, and R^{3a}; when each of R¹, R², and R³ is a hydrogen atom, the hydrogen atom may be substituted with a fluorine atom; when each of R¹, R², and R³ is a substituted or unsubstituted alkyl group, one or two hydrogen atoms on the α-position carbon atom to a carbonyl group may be substituted with a fluorine atom; with fluorine gas in the absence of any solvent and in the presence of one or more acids selected from among trifluoromethanesulfonic acid, methanesulfonic acid, hydrofluoric acid, sulfuric acid, trifluoroacetic acid, boron trifluoride, and sulfonated polymers.

### Best Mode for Carrying Out the Invention

Examples of the alkyl groups in formula (1) include C₁₋₂₀ alkyl groups, preferably C₁₋₁₀ alkyl groups. Specific examples include a methyl group, an ethyl group, an n-propyl group, an isopropyl group, an n-butyl group, a pentyl group, and a hexyl group. Examples of the substituents in the alkyl groups which may be substituted include a halogen atom, a hydroxyl group, a cyano group, an aryl group, an acyl group, an alkoxy group, an aryloxy group, an acyloxy group, an alkoxycarbonyl group, an aryloxycarbonyl group, an alkanesulfonyl group, an arylsulfonyl group, and an acylamino group.

Examples of the aryl groups include C₆₋₁₄ aryl groups, preferably C₆₋₁₀ aryl groups. Preferable examples include a phenyl group and a naphthyl group. Examples of the substituents in the aryl groups which may be substituted include an alkyl group, a halogen atom, a cyano group, a nitro group, an acyl group, an acyloxy group, an alkoxycarbonyl group, an aryloxycarbonyl group, an alkanesulfonyl group, and an arylsulfonyl group. An aryl moiety of an aryloxy group, an aryloxycarbonyl group, and an arylsulfonyl group may be substituted with the similar substituents for the aryl groups.

Examples of the alkoxy groups include C₁₋₂₀ alkoxy groups, preferably C₁₋₁₀ alkoxy groups. Preferable examples include a methoxy group, an ethoxy group, a propoxy group, an isopropoxy group, and a butoxy group. Examples of the substituents in the alkoxy groups which may be substituted include the similar substituents for the above-described alkyl groups. An alkoxy moiety of an alkoxycarbonyl group may be substituted with the similar substituents for the alkoxy groups.

Examples of the halogen atoms include fluorine, chlorine, bromine, and iodine.

Examples of the acyl groups include aromatic acyl groups such as C₇₋₁₅ aromatic acyl groups and aliphatic acyl groups such as C₁₋₂₀ aliphatic acyl groups. An acyl moiety of an acyloxy group and an acylamino group may be substituted with the similar substituents for the acyl groups. An alkane moiety of an alkanesulfonyl group may be substituted with the similar substituents for the above-described alkyl groups.

Examples of the cyclic structure which may be formed by combining R¹, R², and R³ include any one of 3-membered to 20-membered monocyclic structures, bicyclic structure, or polycyclic structure.

In the process for the preparation according to the present invention, the dicarbonyl compounds represented by formula (1) serving as starting materials are easily available industrially or easily synthesized through a customary synthesis method. In the present invention, the dicarbonyl compounds which should be used as starting materials are selected from the dicarbonyl compounds which have a characteristic of liquid at room temperature. Examples of the dicarbonyl compounds represented by formula (1) include the following compounds. In the formulas, Ph represents a phenyl group.
CH₃COCH₂COCH₃, CH₃COCH₂COC₂H₅, CH₃COCH₂COC₅H₁₁, CH₃COCH(CH₃) COCH₃, CH₃COCHFCOCH₃, CH₃COCHClCOCH₃, ClCH₂COCH₂COCH₃, CF₃COCH₂COCH₃, CF₃COCH₂COCF₃, CF₃COCHFCOCF₃, 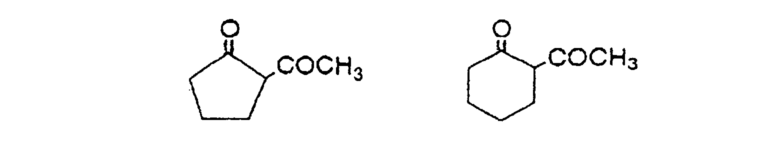 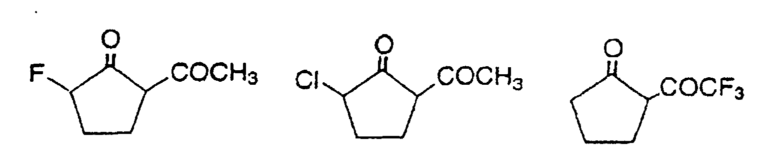 HCOCH₂COOCH₃, HCOCH₂COOC₂H₅, CH₃COCH₂COOC₂H₅, CH₃COCH₂COOC₃H₇, CH₃COCH(CH₃)COOCH₃, CH₃COCH₂COOCH₂Ph, CH₃COCHFCOOC₂H₅, CH₃COCHClCOOC₂H₅,

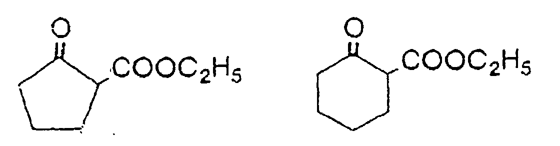

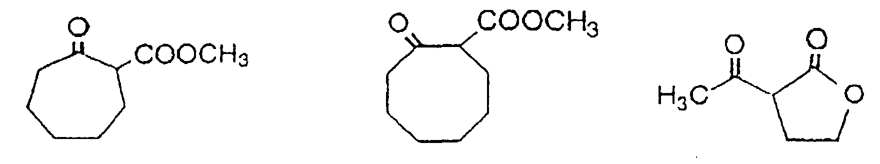

The hydrogen atom which is fluorinated through the method of the present invention is a hydrogen atom on the α-position carbon atom to a carbonyl group, and a hydrogen atom on the carbon atom between two carbonyl groups is most easily fluorinated. The method of the present invention is useful as a process for the preparation of a monofluoro species, although the method also produces a difluoro species which is obtained at low yield. When there are two hydrogen atoms at the carbon atom between two carbonyl groups, the two hydrogen atoms are fluorinated to form the corresponding difluoro species. There is also formed a compound in which a hydrogen atom at either of the carbon atoms located outside the two carbonyl groups is fluorinated.

Preferable examples of R¹ include a group represented by R^{1b}CH₂-, wherein R^{1b} represents a hydrogen atom, a substituted or unsubstituted alkyl group, or a substituted or unsubstituted aryl group.

Preferable examples of R² include a hydrogen atom. Preferable examples of R³ include -OR^{3b}, wherein R^{3b} represents a substituted or unsubstituted alkyl group, or a substituted or unsubstituted aryl group.

The corresponding preferable cases are shown by the following reaction schemes. 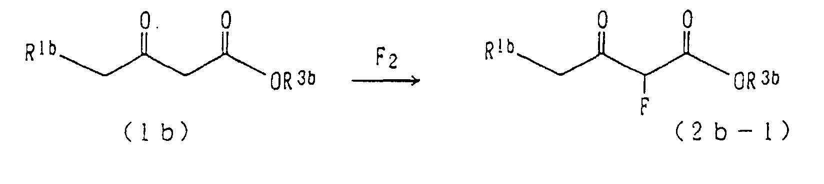 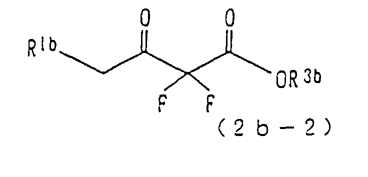 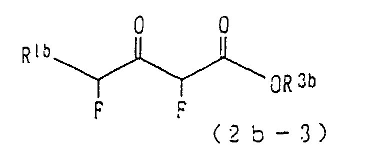 (wherein R^{1b} and R^{3b} have the same meanings as described above).

In the present invention, compound (2b-1) is obtained at high yield, and therefore the process of the invention is particularly useful for the preparation of this compound (2b-1).

The fluorine gas used in the present invention is preferably used after diluted with an inert gas to form a mixture of fluorine gas and an inert gas, since the fluorine gas generally has strong reactivity when used as is. Examples of the inert gas used in dilution include nitrogen, helium, argon, and carbon dioxide, and the mixture gas is preferably used so that the proportion of the inert gas to the total volume of the mixture is regulated to fall within 99.9-50% by volume.

The amount of fluorine gas used is not univocally determined, since it varies depending on the introduction method, reaction temperature, reactor, etc. The amount required to substantially consume the dicarbonyl compounds represented by formula (1) serving as starting materials may be used as a yardstick for the amount of fluorine gas.

Examples of the acid used in the present invention include one or more acids selected from among trifluoromethanesulfonic acid, methanesulfonic acid, hydrofluoric acid, sulfuric acid, trifluoroacetic acid, boron trifluoride, and sulfonated polymers, and trifluoromethanesulfonic acid or hydrofluoric acid is preferably used in view of yield, selectivity, and economy.

These acids may be used in an amount of 1-100 wt.% based on the dicarbonyl compounds (1) serving as starting materials, and the amount is preferably 5-20 wt.% in view of economy and effects of addition.

Reactions in the present invention may be performed at reaction temperatures between -80°C and 100°C, preferably between -20°C and 40°C, in order to effectively proceed reaction and obtain a target compound at high yield.

The resultant fluorinated dicarbonyl compounds may be purified through a customary method such as distillation.

### Examples

The present invention will next be described by way of examples, which should not be construed as limiting the invention.

### Example 1

Methyl 3-oxopentanoate (1a) (2.60 g, 20 mmol) and trifluoromethanesulfonic acid (0.301 g, 10.5 wt.%) were stirred in an ice bath at 0°C. Into the mixture, fluorine gas diluted to have a concentration of 10% by volume with nitrogen gas was passed at a flow rate of 25 ml/minute with stirring. Fluorine gas was used in an amount of 1120 ml (50 mmol).

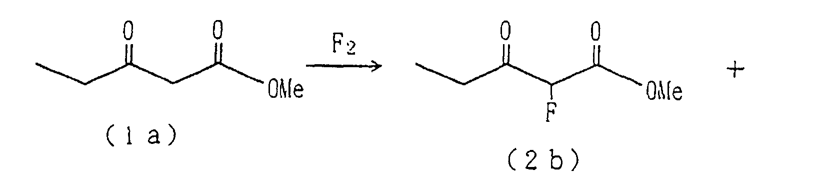

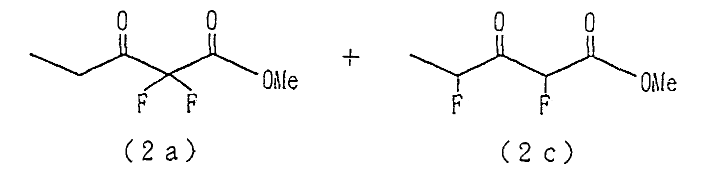

After reaction, the resultant mixture was quantitatively determined by gas chromatography (column: PEG 20M chromosorb WAW, product of GL Science Co., 2 m, column temperature 100°C), to thereby obtain the following yields: methyl 2,2-difluoro-3-oxopentanoate (2a) 7.5%, methyl 2-fluoro-3-oxopentanoate (2b) 82.5%, and methyl 2,4-difluoro-3-oxopentanoate (2c) 8.5%.

### Comparative Example 1

Fluorine gas diluted to have a concentration of 10% by volume with nitrogen gas was passed at a flow rate of 25 ml/minute into methyl 3-oxopentanoate (1a) (2.60 g, 20 mmol) while stirring in an ice bath at 0°C. Fluorine gas was used in an amount of 1120 ml (50 mmol).

After reaction, the resultant mixture was quantitatively determined by gas chromatography, to thereby obtain the following yields: starting material (1a) 15.2%, methyl 2,2-difluoro-3-oxopentanoate (2a) 13.5%, methyl 2-fluoro-3-oxopentanoate (2b) 55.2%, and methyl 2,4-difluoro-3-oxopentanoate (2c) 16.1%.

### Examples 2 to 12

The procedure of Example 1 was performed except that the acids specified in Table 1 and Table 2 were used, to thereby manufacture compound (2a), compound (2b), and compound (2c). Similar to Example 1, fluorine gas was diluted with nitrogen gas.

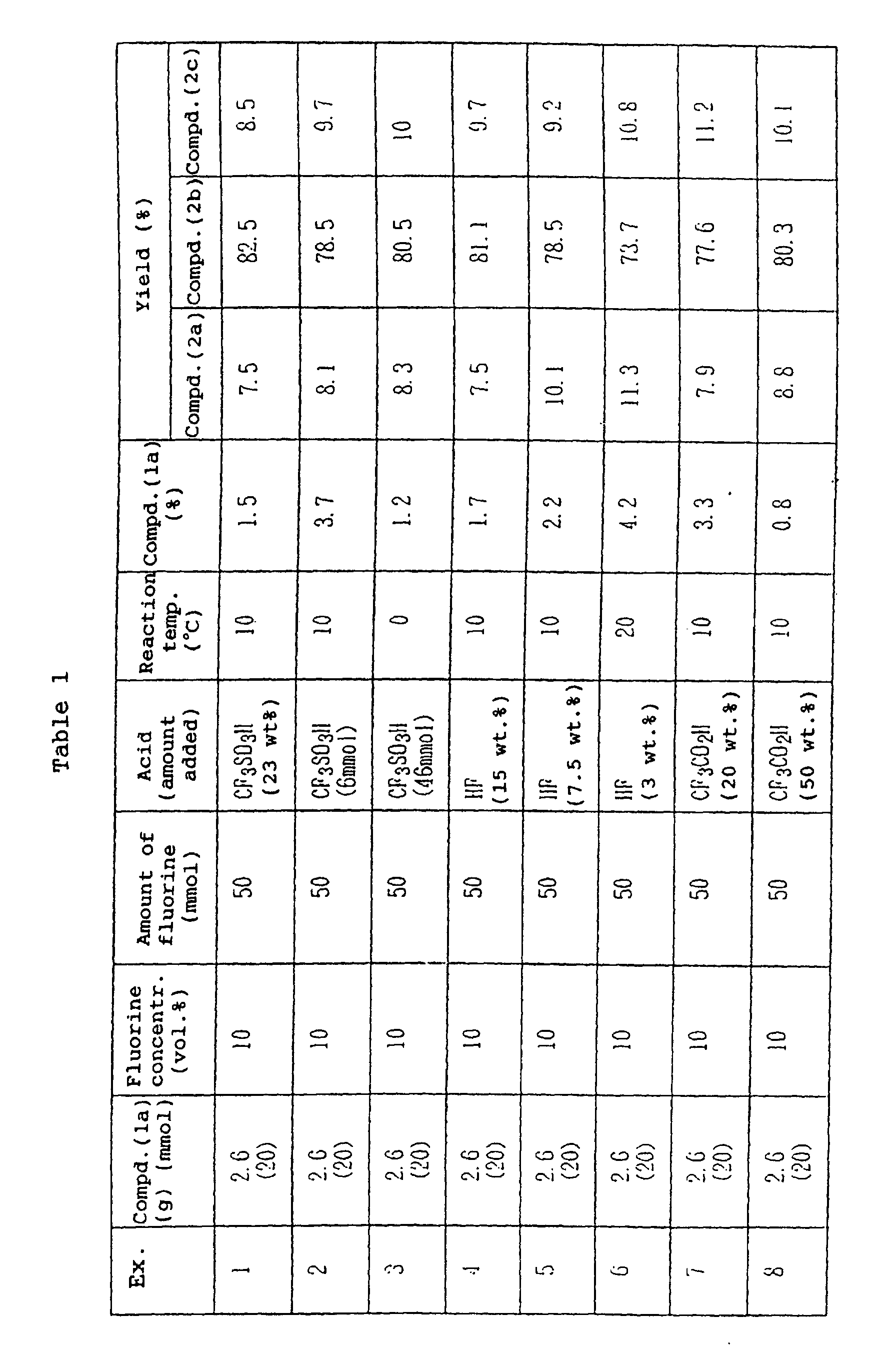

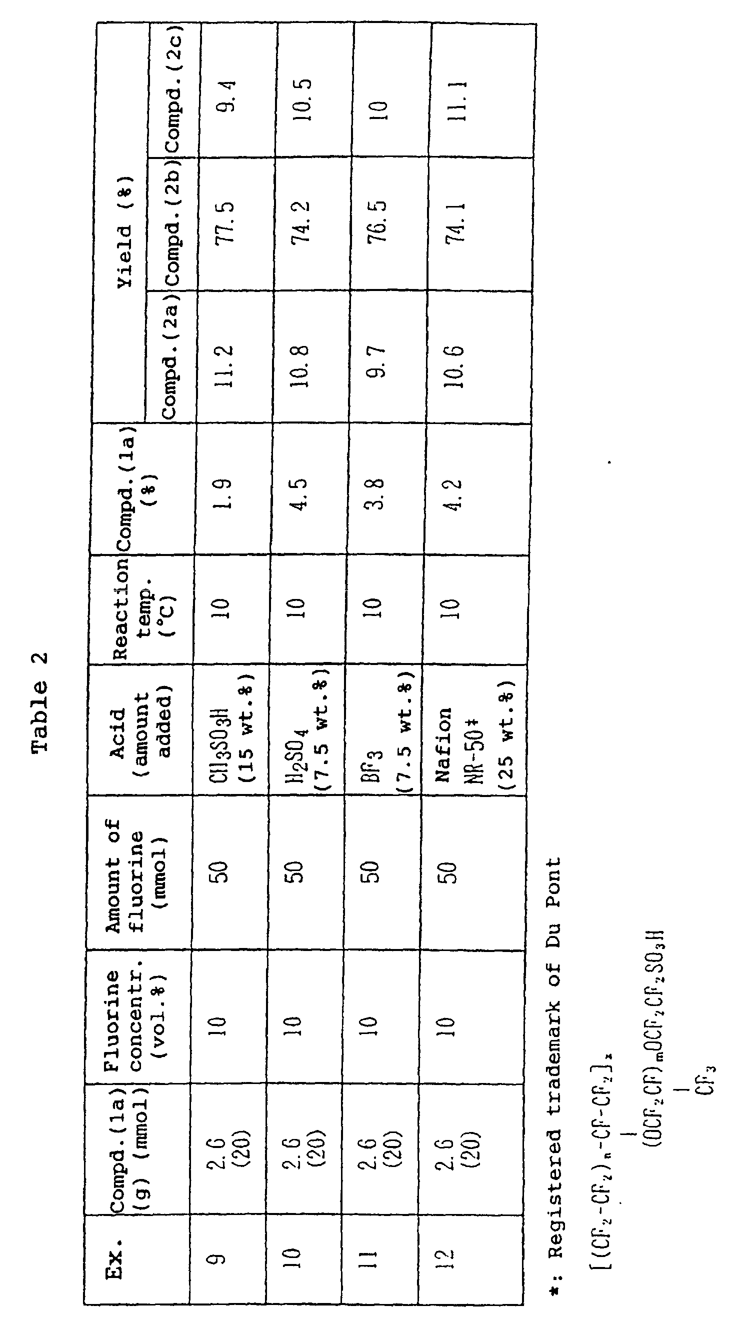

### Examples 13 to 16

The procedure of Example 1 was performed except that the starting materials, acids, etc. specified in Table 3 were used, to thereby manufacture fluorinated dicarbonyl compounds.

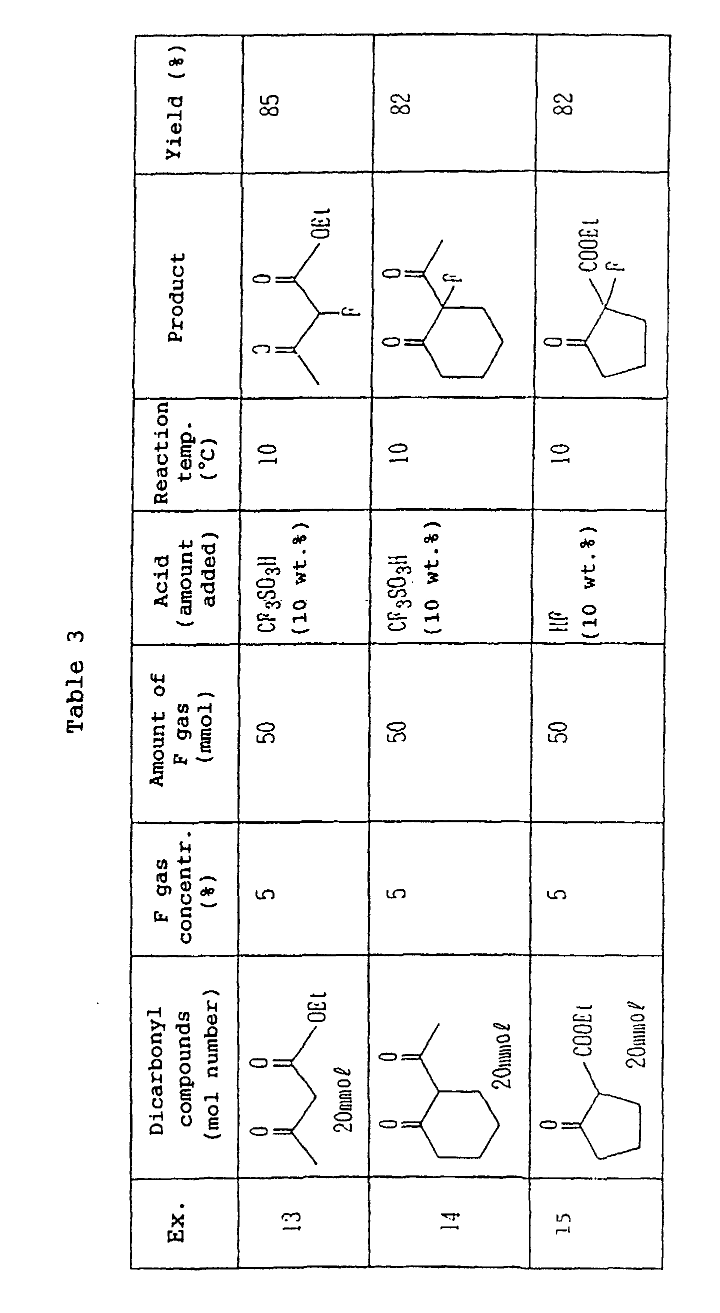

### Industrial Applicability

The present invention enables to manufacture target fluorinated dicarbonyl compounds without use of any solvent, thus the scale of a reactor can be smaller. By adding specific acids, yield and selectivity of target compounds are enhanced compared with those obtained when reaction is performed without any solvent and without adding any acid. Furthermore, these effects advantageously reduces the manufacturing costs of the target compounds.

## Claims

1. A process for manufacturing fluorinated dicarbonyl compounds represented by formula (2) 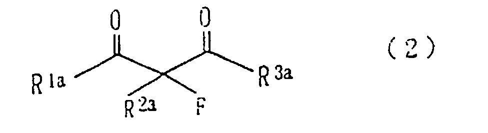 wherein R^{1a} represents a hydrogen atom, a substituted or unsubstituted C₁₋₂₀ alkyl group or a substituted or unsubstituted C₆₋₁₄ aryl group; R^{2a} represents a hydrogen atom, a halogen atom, a substituted or unsubstituted C₁₋₂₀ alkyl group or a substituted or unsubstituted C₆₋₁₄ aryl group; R^{3a} represents a hydrogen atom, a substituted or unsubstituted C₁₋₂₀ alkyl group, a substituted or unsubstituted C₆₋₁₄ aryl group, a substituted or unsubstituted C₁₋₂₀ alkoxy group, or a substituted or unsubstituted C₆₋₁₄ aryloxy group; and at least two of R^{1a}, R^{2a}, and R^{3a} may be combined together to form a portion of a cyclic structure via an optional hetero atom, by reacting dicarbonyl compounds represented by formula (1) 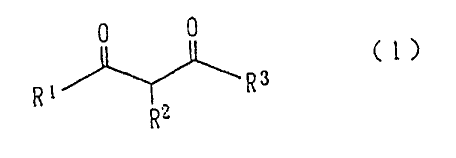 wherein R¹, R², and R³ respectively have the same meanings as R^{1a}, R^{2a}, and R^{3a}; when each of R¹, R², and R³ is a hydrogen atom, the hydrogen atom may be substituted with a fluorine atom; when each of R¹, R², and R³ is a substituted or unsubstituted C₁₋₂₀ alkyl group, one or two hydrogen atoms on the α-position carbon atom to a carbonyl group may be substituted with a fluorine atom) with fluorine gas in the absence of any solvent and in the presence of one or more acids selected from among trifluoromethanesulfonic acid, methanesulfonic acid, hydrofluoric acid, sulfuric acid, trifluoroacetic acid, boron trifluoride, and sulfonated polymers.

2. The process according to claim 1, wherein the amount of the acid is 1 to 100% by weight with respect to the dicarbonyl compound.

3. The process according to claim 2, wherein the amount of the acid is 5 to 20% by weight with respect to the dicarbonyl compound.

## Patentansprüche

1. Verfahren zur Herstellung fluorierter Dicarbonylverbindungen, dargestellt durch die Formel (2) 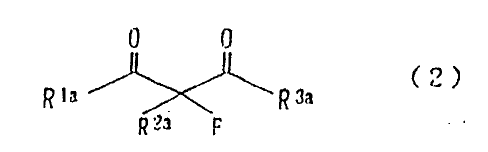 wobei R^{1a} ein Wasserstoffatom darstellt, eine substituierte oder nicht substituierte C₁₋₂₀ Alkylgruppe oder eine substituierte oder nicht substituierte C₆₋₁₄ Arylgruppe; R^{2a} ein Wasserstoffatom darstellt, ein Halogenatom, eine substituierte oder nicht substituierte C₁₋₂₀ Alkylgruppe oder eine substituierte oder nicht substituierte C₆₋₁₄ Arylgruppe; R^{3a} ein Wasserstoffatom darstellt, eine substituierte oder nicht substituierte C₁₋₂₀ Alkylgruppe oder eine substituierte oder nicht substituierte C₆₋₁₄ Arylgruppe, eine substituierte oder nicht substituierte C₁₋₂₀ Alkoxygruppe oder eine substituierte oder nicht substituierte C₆₋₁₄ Aryloxygruppe; und mindestens zwei der R^{1a}, R^{2a} und R^{3a} kombiniert werden können, um einen Teil einer zyklischen Struktur über ein optionales Heteroatom zu bilden durch Reaktion von Dicarbonylverbindungen, dargestellt durch die Formel (1) 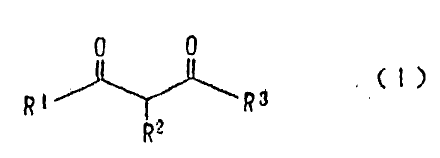 wobei R¹, R² bzw. R³ die gleiche Bedeutung wie R^{1a}, R^{2a} und R^{3a} besitzen; wenn jedes der R¹, R² und R³ ein Wasserstoffatom ist, kann das Wasserstoffatom durch ein Fluoratom substituiert werden; wenn jedes der R¹, R² und R³ eine substituierte oder nicht substituierte C₁₋₂₀ Alkylgruppe ist, können ein oder zwei Wasserstoffatome an dem in der α-Position befindlichen Kohlenstoffatom einer Carbonylgruppe durch ein Fluoratom substituiert werden; mit Fluorgas bei Abwesenheit von Lösungsmittel und bei Anwesenheit von einer oder mehreren Säuren, ausgewählt unter Trifluoromethansulfonsäure, Methansulfonsäure, Fluorwasserstoffsäure, Schwefelsäure, Trifluoressigsäure, Bortrifluorid und sulfonierte Polymere.

2. Verfahren nach Anspruch 1, wobei die Menge der Säure 1 bis 100 Gew.-% in Bezug auf die Dicarbonylverbindung beträgt.

3. Verfahren nach Anspruch 2, wobei die Menge der Säure 5 bis 20 Gew.-% in Bezug auf die Dicarbonylverbindung beträgt.

## Revendications

1. Procédé pour préparer des composés dicarbonylés fluorés représentés par la formule (2) 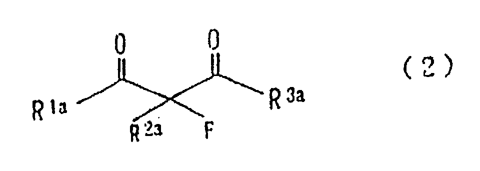 dans lequel R^{1a} représente un atome d'hydrogène, un groupe alkyle C₁₋₂₀ substitué ou non substitué ou un groupe aryle C₆₋₁₄ substitué ou non substitué ; R^{2a} représente un atome d'hydrogène, un atome d'halogène, un groupe alkyle C₁₋₂₀ substitué ou non substitué ou un groupe aryle C₆₋₁₄ substitué ou non substitué ; R^{3a} représente un atome d'hydrogène, un groupe alkyle C₁₋₂₀ substitué ou non substitué, un groupe aryle C₆₋₁₄ substitué ou non substitué, un groupe alkoxy C₁₋₂₀ substitué ou non substitué ou un groupe aryloxy C₆₋₁₄ substitué ou non substitué ; et au moins deux de R^{1a}, R^{2a} et R^{3a} peuvent être combinés ensemble pour former une partie d'une structure cyclique via un hétéroatome optionnel en faisant réagir les composés dicarbonylés représentés par la formule (1) 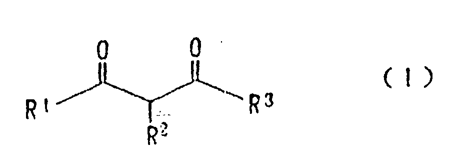 dans lequel R¹, R² et R³ ont respectivement la même signification que R^{1a}, R^{2a} et R^{3a} ; si chacun de R¹, R² et R³ est un atome d'hydrogène, l'atome d'hydrogène peut être substitué par un atome de fluor ; si chacun de R¹, R² et R³ est un groupe alkyle C₁₋₂₀ substitué ou non substitué, un ou deux atomes d'hydrogène sur l'atome de carbone en position α d'un groupe carbonyle peuvent être substitués par un atome de fluor; avec du gaz fluoré en l'absence de tout solvant et en présence d'un ou de plusieurs acides sélectionnés parmi l'acide trifluorométhanesulfonique, l'acide méthanesulfonique, l'acide fluorhydrique, l'acide sulfurique, l'acide trifluoroacétique, le trifluorure de bore et les polymères sulfonés.

2. Procédé selon la revendication 1, dans lequel la quantité d'acide est de 1 à 100 % en poids par rapport au composé dicarbonylé.

3. Procédé selon la revendication 2, dans lequel la quantité d'acide est de 5 à 20 % en poids par rapport au composé dicarbonylé.
